# EUROPÄISCHE PATENTSCHRIFT

(11) **EP 4 389 022 B1**
(45) Veröffentlichungstag und Bekanntmachung des Hinweises auf die Patenterteilung: **12.08.2026**
(21) Anmeldenummer: 23217844.2
(22) Anmeldetag: 19.12.2023
(51) Int. Cl.: A61B 17/22, A61B 17/32, A61B 17/00

(54) **ULTRASCHALLGENERATOR ZUM ZUFÜHREN EINER ELEKTRISCHEN LEISTUNG UND LITHOTRIPSIEVORRICHTUNG ZUM ZERTRÜMMERN VON KÖRPERSTEINEN**
ULTRASONIC GENERATOR FOR SUPPLYING ELECTRIC POWER AND LITHOTRIPSY APPARATUS FOR CRUSHING CALCULI
GÉNÉRATEUR À ULTRASONS POUR FOURNIR UNE PUISSANCE ÉLECTRIQUE ET DISPOSITIF DE LITHOTRIPSIE POUR LE BROYAGE DES CALCULS CORPORELS

(30) Priorität: 20.12.2022 DE 102022134509
(43) Veröffentlichungstag der Anmeldung: 26.06.2024
(73) Patentinhaber: Karl Storz SE & Co. KG, 78532 Tuttlingen (DE)
(72) Erfinder: HINDING, Thomas, 78532 Tuttlingen (DE)

(56) Entgegenhaltungen:
- EP-A1- 3 733 313
- EP-B1- 3 082 625
- WO-A1-2015/047628
- ANONYMOUS: "Protecting Devices from ESD Damage - ElectronicsBeliever", 13 April 2016 (2016-04-13), XP093150568, Retrieved from the Internet <URL:https://web.archive.org/web/20160413191215/http:/electronicsbeliever.com/protecting-devices-from-esd-damage/> [retrieved on 20240411]

## Beschreibung

Die Erfindung betrifft einen Ultraschallgenerator zum Zuführen einer elektrischen Leistung zum Zertrümmern von Körpersteinen, wobei dem Ultraschallgenerator eine Sonotrode, ein Ultraschallschwingungsanreger zum Anregen einer Schwingung der mindestens einen Sonotrode und optional eine Krafterzeugungseinrichtung zum Erzeugen einer Kraft zum Bewegen eines Projektils zur Schlaganregung der Sonotrode zuordenbar sind, wobei der Ultraschallschwingungsanreger durch Zuführen einer Wechselspannung mittels des Ultraschallgenerators mit einer Schwingungsfrequenz anregbar ist, und der Ultraschallgenerator eine Messeinrichtung mit mindestens einer Messeinheit zum Messen eines zeitlichen Verlaufs einer Spannung und/oder eines Stroms und eine Steuer- und/oder Regeleinrichtung zum Anpassen einer vom Ultraschallgenerator an den Ultraschallschwingungsanreger zuführbaren elektrischen Leistung aufweist, wobei die Messeinrichtung zumindest einen zu der mindestens einen Messeinheit parallel angeordneten Widerstand und optional einen zur Messeinheit parallel angeordneten Kondensator aufweist. Des Weiteren betrifft die Erfindung eine Lithotripsievorrichtung zum Zertrümmern und/oder Entfernen von Körpersteinen und ein Verfahren zum Betreiben und/oder Regeln einer Lithotripsievorrichtung.

Die Lithotripsie ist ein bekanntes Verfahren zum Zertrümmern von Körpersteinen, welche sich z.B. durch Kondensation und/oder Auskristallisation von Salzen und Eiweißen als sogenanntes Konkrement in Körperorganen, wie beispielsweise in der Blase oder Niere, bilden. Wenn die Körpersteine zu groß für einen natürlichen Abgang sind und Beschwerden verursachen, müssen diese mit einem Lithotripter zerkleinert werden, sodass die zerkleinerten Steine durch natürliche Ausscheidung und/oder mittels einer Saug-Spül-Pumpe entfernt werden können. Die zu zertrümmernden Körpersteine sind häufig inhomogen mit unterschiedlichen Bestandteilen und/oder Festigkeiten aufgebaut.

Um die Steinzertrümmerungsleistung zu verbessern, werden vor allem in der intrakorporalen Lithotripsie Kombinationssysteme eingesetzt, welche zwei verschiedene Anregungs- und/oder Schwingungsquellen kombiniert verwenden. Dazu wird häufig zusätzlich zur konstanten Ultraschallenergie eine intermittierende, ballistische Schockwellenenergie zugeführt. Dies kann beispielsweise mittels eines ballistischen Antriebs mit Elektromagneten erfolgen, bei dem ein Prallkörper mittels der Elektromagneten beschleunigt wird und auf ein Horn und/oder den Sonotrodenkopf aufschlägt. Bei pneumatischen Lithotriptern wird stattdessen das Projektil innerhalb eines Beschleunigungsrohres durch Zufuhr von Druckluft beschleunigt und die kinetische Energie des Projektils wird über einen elastischen Stoß auf das proximale Ende der Sonotrode und weiter auf deren distales Ende zum Fragmentieren eines Körpersteins übertragen. In einer kombinierten Lithotripsievorrichtung kann üblicherweise eine Pneumatikeinheit vorteilhaft für das Zertrümmern von harten und eine Ultraschalleinheit zum Zertrümmern von weichen Steinen eingesetzt werden.

Für einen effektiven und stabilen Betrieb der Sonotrode in Resonanz ist es allgemein erforderlich, dass alle Komponenten des Lithotripsiesystem von der Sonotrode über den Ultraschallwandler mit dem Horn, dem Übertrager und dem Ultraschallgenerator aufeinander abgestimmt sind. Zur Regelung der Ultraschalleinheit wird üblicherweise die Spannung und der Strom an den Piezoelementen des Ultraschallwandlers detektiert, aus den beiden Sinussignalen werden mittels eines Komparators Rechtecksignale generiert und diese werden abgeglichen. Die Betriebsfrequenz, üblicherweise um 27 kHz, wird beim Betrieb des Ultraschallgenerators so lange nachgeregelt, bis die Rechtecksignale von Strom und Spannung in Phase sind. Durch Erwärmung und mechanische und/oder elektronische Störungen muss die Frequenz mittels des Ultraschallgenerators permanent nachgeregelt werden, um diese in Phase zu halten und einen optimalen Betrieb der Sonotrode zu gewährleisten. Bei einem kombinierten Betrieb schlägt das ballistisch und/oder pneumatisch angetriebene Projektil auf ein distalseitiges Anschlagselement und/oder die Sonotrode auf und dieser mechanische Schlag wird auch auf die Piezoelemente des Ultraschallwandlers übertragen, welche daraufhin eine sehr hohe Spannung erzeugen. Dadurch werden die Messung von Spannung und Strom sowie die Bestimmung der Phasenverschiebung zur Regelung der Frequenz im Ultraschallgenerator gestört und/oder sind nicht mehr durchführbar. Aufgrund des Schlages des Projektils nicht nur auf die Sonotrode, sondern auch auf das die Ultraschallwellen erzeugende Piezoelement, wird die dadurch erzeugte sehr hohe Spannung des Piezoelementes rückübertragen und induziert eine Spannung im Ultraschallgenerator, welche die Resonanzfrequenz des Ultraschallgenerators stört. Dementsprechend wird gleichzeitig die Zertrümmerungsleistung der Sonotrode mittels der Ultraschallanregung aufgrund der Schlaganregung unerwünscht reduziert.

Um eine Störung des Ultraschallgenerators durch den mechanischen Aufschlag eines Projektils zu reduzieren, ist es allgemein bekannt, einen Ultraschallgenerator bei einer konstanten Frequenz zu betreiben. Dies führt jedoch bei Erwärmung, mechanischen und/oder elektronischen Störungen sowie einer Verstimmung des aufeinander abgestimmten Lithotripsiesystems zu Leistungsverlusten. Um diese zu kompensieren, kann die elektrische Leistung erhöht werden, welche den Piezoelementen des Ultraschallwandlers zugeführt wird. Dadurch kann zwar der Leistungsverlust teilweise kompensiert werden, aber der Wirkungsgrad der Steinzertrümmerung bleibt verringert.

Aus der DE 10 2018 101 215 A1 ist eine Vorrichtung zur Zertrümmerung eines Körpersteins mit einer ersten Antriebseinrichtung zur periodischen Auslenkung und eine zweite Antriebseinrichtung zur impulsförmigen Auslenkung einer Sonde bekannt, bei welcher mittels eines als Tiefpassfilter ausgebildeten Übertragungsbereichs zur frequenzselektiven Übertragung von Schwingung eine Schädigung der ersten Antriebseinrichtung durch Entkopplung von der zweiten Antriebseinrichtung reduziert wird. Der Übertragungsbereich ist als Querschnittssprung und/oder Querschnittsverjüngung ausgestaltet. Die Querschnittsverjüngung ist in Übertragungsrichtung von der ersten Antriebseinrichtung auf ein Schwingteil der ersten Antriebseinrichtung ausgebildet, welches auf die Sonde einwirkt, wodurch eine Reflexion eines möglichst großen Anteils der impulsförmigen Schwingung der zweiten Antriebseinrichtung erfolgt. Nachteilig hierbei ist, dass im Bereich der Querschnittsverjüngung sowohl in Längsrichtung der Sonde als auch quer zur Sonde ein Abstand und/oder Freiraum notwendig ist, welcher den Bauraum vergrößert oder zumindest eine effiziente Nutzung des vorhandenen Bauraums verhindert.

Das Patent EP 3 082 625 B1 offenbart eine medizinische Vorrichtung zum Schneiden und Koagulieren von Gewebe mittels Ultraschall sowie die dazugehörige Steuereinrichtung. Weiterhin ist es bekannt, elektronische Schaltkreise durch Suppressordioden vor Spannungsspitzen zu schützen (https://web.archive.org/web/20160413191215/http://electronics-believer.com/protecting-devices-from-esd-damage).

Aus der Offenlegungsschrift EP 3 733 313 A1 ist eine Lithotripsievorrichtung bekannt, deren Sonotrode sowohl von einem Ultraschallgenerator als auch von einem linear beweglichen Projektil angeregt wird.

Weiter nachteilig bei bekannten Gegenmaßnahmen ist, dass üblicherweise nur die gegenseitige Beeinflussung zwischen einer ballistischen Einheit und der Ultraschalleinheit direkt minimiert wird, diese jedoch nicht im Falle einer nachteiligen Rückübertragung auf den Ultraschallgenerator wirken und eine Regelbarkeit der Betriebsfrequenz des Ultraschallgenerators ermöglichen.

Aufgabe der Erfindung ist es, den Stand der Technik zu verbessern.

Gelöst wird die Aufgabe durch einen Ultraschallgenerator zum Zuführen einer elektrischen Leistung zum Zertrümmern von Körpersteinen, wobei dem Ultraschallgenerator eine Sonotrode, ein Ultraschallschwingungsanreger zum Anregen einer Schwingung der mindestens einen Sonotrode und eine Krafterzeugungseinrichtung zum Erzeugen einer Kraft zum Bewegen eines Projektils zur Schlaganregung der Sonotrode zuordenbar sind, wobei der Ultraschallschwingungsanreger durch Zuführen einer Wechselspannung mittels des Ultraschallgenerators mit einer Schwingungsfrequenz anregbar ist, und der Ultraschallgenerator eine Messeinrichtung mit mindestens einer Messeinheit zum Messen eines zeitlichen Verlaufs einer Spannung und/oder eines Stroms und eine Steuer- und/oder Regeleinrichtung zum Anpassen einer vom Ultraschallgenerator an den Ultraschallschwingungsanreger zuführbaren elektrischen Leistung aufweist, wobei die Messeinrichtung zumindest einen zu der mindestens einen Messeinheit parallel angeordneten Widerstand und optional einen zur Messeinheit parallel angeordneten Kondensator aufweist, und die Messeinrichtung parallel zu der mindestens einen Messeinheit mindestens eine Suppressordiode zum Unterdrücken von Überspannungen aufweist, wobei die Steuer- und/oder Regeleinrichtung eine Phasenregelschleife-Einheit aufweist, sodass die zuführbare elektrische Leistung und/oder die Frequenz mittels der Steuer- und/oder Regeleinrichtung derart anpassbar ist oder sind, dass eine Phasenverschiebung der zeitlichen Verläufe der Spannung und des Stroms Null ist oder einen vorgegebenen Wert aufweist und bei einer Schlaganregung der Sonotrode mittels des Projektils die zuvor bestimmte Frequenz und/oder Resonanzfrequenz der zuführbaren elektrischen Leistung vorgebbar ist.

Somit wird ein Ultraschallgenerator für eine Lithotripsievorrichtung bereitgestellt, bei dem aufgrund der spezifisch ausgebildeten Messeinrichtung rückübertragene Spannungsspitzen erzeugt von einem Piezoelement in dem zugeordneten Ultraschallschwingungsanreger, beispielsweise verursacht durch eine Schlaganregung eines Projektils, unterdrückt werden. Dadurch wird eine Messung eines zeitlichen Verlaufs einer Spannung und/oder eines Stromes trotz mechanischen und/oder elektrischen Störungen mittels der Messeinrichtung und eine Anpassung der vom Ultraschallgenerator an den Ultraschallschwingungsanreger zugeführten elektrischen Leistung und/oder Frequenz ermöglicht. Folglich wird die Messeinrichtung vor Überspannung erzeugt von dem Ultraschallschwingungsanreger und/oder Piezoelement geschützt. Dadurch ist ein Ultraschallgenerator für eine kombinierte Lithotripsievorrichtung mit einer störungsfrei funktionierenden Ultraschalleinheit und einer ballistischen und/oder pneumatischen Einheit zur Schlaganregung bereitgestellt, bei welcher die Zertrümmerungsleistung der Schlaganregung maximiert werden kann, ohne die Funktion des Ultraschallgenerators und/oder der Ultraschalleinheit zu beeinträchtigen.

Dadurch, dass der Ultraschallgenerator die Messeinrichtung aufweist, werden Spannung und/oder Strom auf der Primärseite des Übertragers zu dem Ultraschallschwingungsanreger gemessen, wodurch die Messeinheit der Messeinrichtung galvanisch von dem Piezoelement oder den Piezoelementen des Ultraschallschwingungsanregers getrennt ist und eine Rückübertragung auf den Ultraschallgenerator zumindest gemindert ist.

Aufgrund der Dämpfung des gemessenen Signales durch den Widerstand und eine Entstörung mittels der Suppressordiode, welche störende Spannungsspitzen eliminiert oder zumindest minimiert, ist die Messeinrichtung weitgehend auch bei mechanischen und/oder elektrischen Störungen und/oder einer Schlaganregung mittels eines Projektils verlässlich betreibbar. Dadurch können kurzzeitig auftretende Spannungsspitzen, wie beispielsweise von 1.000 V, erzeugt von dem das Piezoelement des Ultraschallwandlers aufgrund einer Schlaganregung mittels der Suppressordiode der Messeinrichtung zumindest teilweise weggenommen und dadurch die parallel geschaltete Messeinheit geschützt werden. Ist trotz dieses Schutzes der Messeinrichtung bei sehr starken Störungen eine Messung von Spannung und/oder Strom und dem Bestimmen des Vorliegens beider in Phase nicht möglich, so ist dieser kurzzeitige Zustand mittels der Steuer- und/oder Regeleinrichtung überbrückbar, indem bekannte Werte der Resonanzfrequenz verwendet werden. Dadurch arbeitet der Ultraschallschwingungsanreger im Wesentlichen in Resonanz und gibt eine hohe Zertrümmerungsleistung bei optimalem Wirkungsgrad ab.

Ein wesentlicher Gedanke der Erfindung beruht darauf, dass eine mechanische und/oder elektrische Störung des Ultraschallschwingungsanregers und/oder die negative Beeinflussung des Piezoelemente des Ultraschallschwingungsanregers durch die Schlaganregung eines Projektils gerade nicht durch konstruktive, mechanische Ausgestaltung des Ultraschallschwingungsanregers, sondern durch elektronisches Rausfiltern und/oder Dämpfen der Störungen und/oder Überspannungsimpulse mittels der Messeinrichtung selbst des Ultraschallgenerators erfolgt und anschließend die derart erhaltenen Messsignale mittels der Steuer- und/oder Regeleinrichtung analysierbar und optimal zum Betrieb des Ultraschallgenerators verwendbar sind. Damit ist sichergestellt, dass der Ultraschallwandler in Resonanz arbeitet und durch eine Schlaganregung mittels eines Projektils nicht in seiner Zertrümmerungsleistung negativ beeinflusst wird. Folglich wird auch in einem kombinierten Lithotripsiesystem eine optimale Leistungszuführung zur Sonotrode und jeweils eine hohe Zertrümmerungsleistung der Schwingungsanregung mittels des Ultraschallwandlers und der Schlaganregung mittels des Projektils erzielt.

### Folgendes Begriffliche sei erläutert:

Bei einer "Lithotripsievorrichtung" (auch "Lithotripter" genannt) handelt es sich insbesondere um eine Vorrichtung zum Zertrümmern von Körpersteinen durch Stöße, Stoßwellen, Verformungswellen und/oder Schwingungswellen. Unter einer Lithotripsievorrichtung werden insbesondere verschiedene Bestandteile, Bau- und/oder Funktionskomponenten eines Lithotripters verstanden. Die Lithotripsievorrichtung kann einen Lithotripter vollständig oder teilweise ausbilden. Bei einer Lithotripsievorrichtung kann es sich insbesondere um eine intrakorporale oder extrakorporale Lithotripsievorrichtung handeln. Im Falle einer intrakorporalen Lithotripsievorrichtung kann diese zusätzlich eine Spül-/Saugpumpe aufweisen. Die Lithotripsievorrichtung kann als Handgerät ausgebildet sein und/oder ein Endoskop aufweisen oder in ein Endoskop eingeschoben werden. Die Lithotripsievorrichtung ist insbesondere autoklavierbar und weist beispielsweise Instrumentenstahl und/oder Kunststoff auf. Die Lithotripsievorrichtung kann weitere Komponenten, wie ein Steuer- und/oder Versorgungsgerät aufweisen oder diese sind der Lithotripsievorrichtung zugeordnet. Eine Lithotripsievorrichtung ist insbesondere eine kombinierte Lithotripsievorrichtung mit einer ballistischen und/oder pneumatischen Einheit und zuordenbaren Krafterzeugungseinrichtung und mit einem Ultraschallschwingungsanreger. Mittels der ballistischen und/oder pneumatischen Einheit und der zuordenbaren Krafterzeugungseinrichtung wird mittels einer Stoßenergie beim Anschlagen eines Projektils an einem distalseitigen Anschlagselement insbesondere der Sonotrode direkt oder indirekt eine gezielt geformte Verformungswelle aufgeprägt. Die Verformungswelle bewirkt insbesondere eine translatorische Bewegung der Sonotrode, welche aufgrund der Auslenkung eine Steinzertrümmerung bewirkt. Gleichzeitig wird bei der kombinierten Lithotripsievorrichtung neben dem mechanischen Stoß die Sonotrode zusätzlich insbesondere mittels eines Ultraschallschwingungsanreger in eine Schwingung, insbesondere longitudinale Schwingung und/oder Querschwingung, angeregt. Somit ist die Sonotrode insbesondere als Wellenleiter für die Schwingungswellen erzeugt von dem Ultraschallschwingungsanreger und für die Verformungswellen des Projektils ausgebildet.

Unter "Körpersteinen" (auch "Konkrement" genannt) werden insbesondere alle Steine in einem menschlichen oder tierischen Körper verstanden, welche sich z.B. aus Salzen und Eiweißen durch Kristallisation und/oder Kondensation bilden. Bei Körpersteinen kann es sich beispielsweise um Gallensteine, Harnsteine, Nierensteine und/oder Speichelsteine handeln. Durch Einwirken der Sonotrode und/oder Hohlsonde auf den Körperstein entstehen insbesondere Körpersteinkerne (auch Bohrkerne genannt) und/oder Körpersteinfragmente.

Eine "Trägereinheit" (auch "Handstück" genannt) ist insbesondere ein Hand- und/oder Halteteil der Lithotripsievorrichtung. Bei der Trägereinheit kann es sich insbesondere um eine Handhabe zur manuellen und/oder automatisierten Bedienung und/oder Verbindung der Lithotripsievorrichtung handeln. Eine Trägereinheit kann auch an einem distalen Ende eines Roboterarms angeordnet, verbunden und/oder automatisiert geführt sein. Die Trägereinheit weist insbesondere ein Gehäuse auf. Die Trägereinheit kann auch zwei- oder mehrteilig ausgebildet sein. Beispielsweise kann die Trägereinheit ein separates Gehäuse für eine Pneumatikeinheit und ein separates Gehäuse für den Ultraschallschwingungsanreger aufweisen.

Unter "distalseitig" und "distal" wird eine patientenkörpernahe und somit benutzerferne Anordnung und/oder ein entsprechendes Ende oder Abschnitt verstanden. Dementsprechend wird unter "proximalseitig" oder "proximal" eine benutzernahe und somit patientenkörperferne Anordnung oder ein entsprechendes Ende oder Abschnitt verstanden.

Eine "Sonotrode" ist insbesondere ein Bauteil, welches durch das Einwirken und/oder Einleiten von mechanischen Schwingungen selbst in Schwingung und/oder Resonanzschwingung versetzt wird. Die Sonotrode ist insbesondere als Wellenleiter für die Schwingungswellen erzeugt von dem Ultraschallschwingungsanreger und für die Verformungswellen durch Anschlag des mittels der Krafterzeugungseinrichtung beschleunigten Projektils ausgebildet. Die Sonotrode ist insbesondere mit dem Ultraschallschwingungsanreger und/oder dem Horn direkt oder indirekt verbunden. Beispielsweise ist die Sonotrode in das distalseitige Ende des Horns eingeschraubt. Die Sonotrode weist insbesondere an ihrem proximalen Ende einen Sonotrodenkopf zum Aufnehmen, Weiterleiten und/oder Fokussieren von Ultraschallwellen und an ihrem distalen Ende eine Sonotrodenspitze zum unmittelbaren und/oder mittelbaren Beaufschlagen und/oder Kontaktieren von Körpersteinen auf. Die Sonotrode ist insbesondere derart geformt, dass diese optimal die Schwingungswellen, die Ultraschallschwingung und/die Verformungswellen an ihrem distalen Ende in den Körper, die zu behandelnde Körperregion und/oder direkt auf den zu zertrümmernden Körperstein einleitet. Im Falle der Ultraschallanregung arbeitet die Sonotrode insbesondere im Ultraschallbereich mit einem Frequenzbereich von 20 kHz bis 90 kHz, bevorzugt von 20 kHz bis 34 kHz. Die Sonotrode weist insbesondere Stahl, Titan, Aluminium und/oder Carbon auf. Bei einer Sonotrode handelt es sich insbesondere um eine Sonde, welche beispielsweise stab-, röhren- und/oder schlauchförmig ausgebildet ist. Die Sonotrode kann einstückig oder mehrteilig ausgebildet sein. Die Sonotrode weist insbesondere einen Durchmesser in einem Bereich von 0,5 mm bis 4,5 mm, insbesondere von 0,8 mm bis 3,8 mm, auf.

Ein "Projektil" ist insbesondere ein Körper, welcher innerhalb eines Hohlraums eines Beschleunigungsrohres frei entlang der Beschleunigungsstrecke beweglich ist. Das Projektil ist insbesondere zwischen dem proximalseitigen Anschlagselement und dem distalseitigen Anschlagselement innerhalb des dazwischen angeordneten Hohlraums des Beschleunigungsrohres hin- und zurückbewegbar. Prinzipiell kann das Projektil jegliche Form aufweisen. Beispielsweise kann das Projektil die Form eines Bolzens oder einer Kugel aufweisen. Das Projektil weist insbesondere harten Stahl und/oder schwachmagnetische Eigenschaften auf. Für die freie Beweglichkeit weist das Projektil insbesondere einen etwas geringeren Außendurchmesser als der Durchmesser des Hohlraumes des Beschleunigungsrohres auf. Beispielsweise kann das Projektil einen Außendurchmesser von 8 mm, insbesondere 6 mm, oder 4 mm aufweisen.

Das Projektil kann insbesondere entlang der Beschleunigungsstrecke stetig oder unstetig mittels der Krafterzeugungseinrichtung hin- und/oder herbewegt werden. Bevorzugt wird das Projektil intermittierend und/oder oszillierend zwischen dem proximalseitigen Anschlagselement und dem distalseitigen Anschlagselement hin und her bewegt.

Bei einer "Krafterzeugungseinrichtung" kann es sich prinzipiell um jegliche Art von Einrichtung handeln, welche eine Kraft auf das Projektil und somit eine Bewegung des Projektils bewirkt. Bei der Krafterzeugungseinrichtung kann es sich beispielsweise um eine Vorrichtung handeln, welche mittels Lasers, eines Druckmediums, beispielsweise pneumatisch mithilfe von Druckluft, mittels eines elektromagnetischen Feldes und/oder mittels einer mechanischen Vorrichtung das Projektil beschleunigt. Eine pneumatisch Krafterzeugungseinrichtung kann insbesondere mittels eines Zuführens und/oder Abführens eines Druckmediums eine lineare Bewegung des Projektils im Hohlraum des Beschleunigungsrohres bewirken. Das Druckmedium strömt insbesondere durch mindestens eine proximalseitige Öffnung des Beschleunigungsrohres in den Hohlraum des Beschleunigungsrohres und drückt und beschleunigt das Projektil in distale Richtung.

Ein "Beschleunigungsrohr" ist insbesondere ein länglicher Hohlkörper, dessen Länge eine größere Abmessung aufweist als sein Durchmesser. Das Beschleunigungsrohr weist in seinem Inneren insbesondere einen Hohlraum auf, in dem ein Projektil sich frei in Längsrichtung bewegen kann. Des Weiteren weist das Beschleunigungsrohr insbesondere ein proximales Ende und ein distales Ende auf, welche räumlich abzüglich der Länge des Projektils die maximale Beschleunigungsstrecke festlegen. Das Beschleunigungsrohr ist distalseitig und/oder an seinem distalen Endabschnitt insbesondere zumindest teilweise von dem Horn und/oder einem mit dem Horn verbundenen oder zugeordneten Bolzen umgeben. Bei einer pneumatischen Krafterzeugungseinrichtung weist das Beschleunigungsrohr zumindest eine Öffnung zum Eintritt und/oder Austritt eines Druckmediums, insbesondere Druckluft, auf. Das Beschleunigungsrohr weist als Material insbesondere ein Metall auf.

Ein "Anschlagselement" ist insbesondere ein gewollter Endpunkt der Bewegung des Projektils entlang der Beschleunigungsstrecke innerhalb des Hohlraums des Beschleunigungsrohres, an dem das beschleunigte Projektil gegen das Anschlagselement schlägt, abgebremst und/oder in die Gegenrichtung bewegt wird. Ein distalseitiges Anschlagselement ist insbesondere am und/oder im distalen Ende des Beschleunigungsrohres und/oder innerhalb des Hohlraums in einem Bereich des distalen Abschnittes des Beschleunigungsrohres angeordnet. Das distalseitige Anschlagselement übertragt insbesondere direkt oder indirekt den Stoß des Projektils auf die Sonotrode. Bei dem distalseitigen Anschlagselement kann es sich beispielsweise um eine proximalseitige Wand des Horns, ein Federelement oder um eine proximalseitige Wand eines Halters eines Federelementes handeln. Das proximalseitige Anschlagselement ist insbesondere am und/oder im proximalen Ende des Beschleunigungsrohrs oder innerhalb des Hohlraums in einem proximalen Abschnitt des Beschleunigungsrohrs angeordnet. Bei dem proximalseitigen Anschlagselement kann es sich beispielsweise um eine Wand des Gehäuses, einer Aufnahme für das Beschleunigungsrohr und/oder um ein Federelement handeln.

Ein "Ultraschallgenerator" ist insbesondere ein elektrisches Gerät zum Zuführen einer Wechselspannung an einen Ultraschallschwingungsanreger zum Erzeugen einer Resonanzschwingung in einer Sonotrode. Der Ultraschallgenerator weist insbesondere ein Netzteil, einen Sinusgenerator, eine elektronischen Abtasteinheit und/oder einen Oszillator und/oder einen transformierenden Übertrager der Wechselspannung auf. Mittels des Ultraschallgenerators wird insbesondere eine Wechselspannung mit veränderbarer Amplitude und/oder Frequenz erzeugt.

Der "Sinusgenerator" (auch "Signalgenerator" genannt) ist insbesondere ein elektronisches Gerät, Baugruppe oder Schaltung, welche eine elektrische Spannung mit einem charakteristischen Zeitverlauf erzeugt. Die erzeugte Wechselspannung weist insbesondere eine Frequenz in einem Bereich von 20 kHz bis 90 kHz, bevorzugt von 20 kHz bis 34 kHz, auf. Der Signalgenerator des Ultraschallgenerators ist insbesondere elektrisch mit dem Ultraschallschwingungsanreger verbunden. Die vom Sinusgenerator zugeführte elektrische Leistung bestimmt insbesondere die Amplitude und somit den Schwingungsausschlag der Sonotrode.

Ein "Ultraschallschwingungsanreger" (auch "Schwingungsanreger" genannt) ist insbesondere jegliche Einrichtung zum Erzeugen von Schwingungen im Ultraschallbereich. Der Ultraschallschwingungsanreger ist insbesondere ein Bauteil eines Ultraschallwandlers und/oder Handstückes einer Lithotripsievorrichtung, welcher die zugeführte Wechselspannung mit einer bestimmten Frequenz in eine mechanische Schwingungsfrequenz umsetzt. Der Ultraschallschwingungsanreger ist insbesondere ein elektromechanischer Wandler unter Ausnutzung des piezoelektrischen Effekts. Durch Anlegen der von einem Ultraschallgenerator erzeugten elektrischen Wechselspannung wird eine mechanische Schwingung aufgrund eines Verformens mindestens eines Piezoelementes des Ultraschallwandlers erzeugt. Der Ultraschallwandler weist insbesondere ein Piezoelement oder zwei oder mehrere, bevorzugt gestapelte, Piezoelemente auf. Bevorzugt weist der Ultraschallschwingungsanreger mindestens zwei Piezoelemente auf, wobei zwischen den Piezoelementen ein elektrischer Leiter, beispielsweise eine Kupferscheibe, angeordnet ist. Ein distalseitiges Piezoelement des Ultraschallschwingungsanregers ist insbesondere direkt an einer proximalen Wand eines proximalen Horns angeordnet. Proximalseitig des Piezoelementes oder der Piezoelemente ist insbesondere ein Gegenlager angeordnet.

Ein "Horn" ist insbesondere ein Bauteil, welches zwischen dem Ultraschallschwingungsanreger und/oder einem Piezoelement und der Sonotrode angeordnet ist. Das Horn dient insbesondere dazu, die vom Ultraschallschwingungsanreger erzeugten Ultraschallwellen zur Sonotrode zu übertragen, weiterzuleiten, zu fokussieren und/oder auszurichten. Dazu kann das Horn sich in einer Übertragungsrichtung verjüngen und direkt oder indirekt die Ultraschallwellen auf einen Sondenkopf übertragen. Das Horn kann auch zur Befestigung der Sonotrode verwendet werden. Gleichzeitig dient das Horn insbesondere zusammen mit einem Gegenlager zur beidseitigen mechanischen Halterung des Piezoelementes oder der Piezoelemente. Das Gegenlager ist bevorzugt ein Reflektor für die Ultraschallwellen. Gleichzeitig dienen das Gegenlager und das Horn auch als mechanische Halterung für das Beschleunigungsrohr, welches im Inneren des Gegenlagers und des Horns in Längsrichtung angeordnet ist. Das Gegenlager und/oder das Horn weisen insbesondere einen Hohlraum zur Aufnahme des Beschleunigungsrohres auf.

Eine "Wechselspannung" ist insbesondere eine elektrische Spannung, deren Polarität in regelmäßigen Wiederholungen wechselt.

Eine "Frequenz" ist insbesondere ein Maß dafür, wie schnell bei einem periodischen Vorgang die Wiederholungen aufeinander folgen. Die Frequenz ist insbesondere der Kehrwert der Periodendauer. Die Frequenz gibt insbesondere die Zahl der Perioden der Wechselspannung an, welche in einer Sekunde durchlaufen werden. Eine "Resonanzfrequenz" ist insbesondere eine Frequenz bei Anregung der Sonotrode, bei der die Amplitude stärker wächst als bei Anregung mit benachbarten Frequenzen. Die Resonanzfrequenz ist insbesondere diejenige Frequenz, bei der die Amplitude einer erzwungenen Schwingung maximal wird. Wenn eine Sonotrode mehrere Eigenfrequenzen aufweist, so hat diese insbesondere mehrere lokale Maxima der erzwungenen Amplitude und somit mehrere Resonanzfrequenzen. Die "Betriebfrequenz" des Ultraschallgenerators ist insbesondere diejenige Frequenz, bei welcher eine Phasenverschiebung von Null der Verläufe von Spannung und Strom bestimmt ist oder wird oder ein vorgegebener Wert der Phasenverschiebung vorliegt.

Eine "Messeinrichtung" ist insbesondere ein Gerät, eine Baugruppe und/oder mehrere Bauteile, mit dem oder denen der zeitliche Verlauf der Spannung und/oder des Stroms innerhalb des Ultraschallgenerators messbar ist. Eine Messeinrichtung weist insbesondere eine Messeinheit zum Messen eines zeitlichen Verlaufs der Spannung und/oder des Stromes, einen parallel zur Messeinheit angeordneten Widerstand und zumindest eine parallel angeordnete Suppressordiode zum Unterdrücken von Überspannungen auf. Optional kann die Messeinrichtung einen parallel zur Messeinheit angeordneten Kondensator aufweisen. Die Messeinrichtung misst insbesondere die Spannung direkt, während in einem zweiten Kanal der Spannungsabfall über einen Widerstand, insbesondere einen Shunt-Widerstand, gemessen wird, welches dem Strom entspricht. Ebenso kann mittels einer Strommesszange oder einer Strommessspule als Messeinheit an einer Verbindungsleitung zwischen einem Sinusgenerator und dem Übertrager zum Ultraschallschwingungsanreger innerhalb des Ultraschallgenerators der Strom gemessen werden.

Eine "Suppressordiode" ist insbesondere ein elektronisches Bauelement, welches Strom in einer Richtung passieren lässt und in einer anderen Richtung den Stromfluss sperrt. Eine Suppressordiode dient insbesondere zum Schutz der elektronischen Schaltung der Messeinrichtung vor kurzzeitigen Spannungsimpulsen. Eine Suppressordiode wird insbesondere leitend, wenn eine bauelementspezifische Spannungsschwelle überschritten wird. Hierbei wird insbesondere der Strom des Spannungsimpulses durch Parallelschaltung an dem zu schützenden Bauteil, insbesondere der Messeinheit, vorbeigeführt. Dadurch kann sich insbesondere keine zerstörerische Spannung oberhalb der Durchbruchsspannung der Suppressordiode aufbauen.

Unter einer "Steuereinrichtung" wird insbesondere eine Einrichtung verstanden, welche einen vorgegebenen Wert setzt. Unter einer "Regeleinrichtung" wird insbesondere eine Einrichtung verstanden, welche einen Messwert rückkoppelt und jeweils einen Stellwert einstellt. Somit kann mittels der Steuer- und/oder Regeleinrichtung die optimale und/oder zulässige elektrische Leistung und/oder Frequenz eingestellt und/oder geregelt werden. Die Steuer- und Regeleinrichtung kann eine elektronische Abtasteinheit aufweisen. Eine elektronische Abtasteinheit ist insbesondere ein elektronisches Messgerät, welches eine elektrische Spannung oder mehrere elektrische Spannungen in ihrem zeitlichen Verlauf aufnimmt, digitalisiert und/oder auf einem Bildschirm sichtbar macht. Die elektronische Abtasteinheit weist insbesondere mindestens zwei Kanäle auf. Die elektronische Abtasteinheit weist nicht zwingend einen Bildschirm zum Sichtbarmachen der zeitlichen Verläufe auf, sondern diese Daten können direkt intern innerhalb der Steuer- und/oder Regeleinrichtung und/oder einer Datenspeicher- und/oder Datenverarbeitungseinheit des Ultraschallgenerators aufgenommen und/oder verarbeitet werden. Bei einer elektronischen Abtasteinheit kann es sich um ein Oszilloskop, insbesondere ein digitales Oszilloskop, handeln, wobei das Oszilloskop in dem Ultraschallgenerator integriert oder diesem zugeordnet sein kann.

In einer weiteren Ausführungsform des Ultraschallgenerators ist die mindestens eine Messeinheit eine Messspule und die Messeinrichtung weist den Kondensator zum Ausbilden eines Parallelschwingkreises und den Widerstand zum Dämpfen eines zeitlichen Verlaufs der Spannung und/oder des Stroms auf.

Dadurch, dass der Kondensator parallel zu der Messeinheit und/oder der Messspule zum Messen des Stromes geschaltet ist und die Größe der Kapazität des Kondensators dementsprechend ausgewählt ist, bilden die Messeinheit und/oder Messspule und der Kondensator einen Schwingkreis von insbesondere etwa 27 kHz aus, welches der Resonanzfrequenz der Sonotrode entspricht.

Ein "Kondensator" ist insbesondere ein passives elektrisches Bauteil, mit welchem elektrische Ladung in einem elektrischen Feld speicherbar ist. Die gespeicherte Ladung pro Spannung wird insbesondere als elektrische Kapazität bezeichnet. In einem Wechselstromkreis wirkt der Kondensator insbesondere als Wechselstromwiderstand mit einem frequenzabhängigen Impedanzwert. Der Kondensator weist insbesondere zwei Elektroden als elektrisch leitfähige Flächen auf, welche mit einem Dielektrikum als isolierendes Material voneinander getrennt sind. Die Größe der Kapazität des Kondensators wird insbesondere durch die Fläche der Elektroden, das Material des Dielektrikums und/oder den Abstand der Elektroden zueinander bestimmt.

Um den Strom mittels der Messeinrichtung in einfacher Weise zu bestimmen, ist die mindestens eine Messeinheit ein Messwiderstand zum Messen des zeitlichen Verlaufs des Stroms.

Somit wird ein Nebenwiderstand zu einem Teil des Stromkreises parallelgeschaltet. Bei einem Messwiderstand kann es sich insbesondere um einen Shunt-Widerstand handeln.

Ein "Messwiderstand" (auch "Nebenwiderstand" oder "Shunt-Widerstand" genannt) ist insbesondere ein elektrisch leitendes Bauelement, das zu einem Teil eines Stromkreises parallelgeschaltet ist, um einen elektrischen Strom von diesem Teil abzuleiten. Ein Messwiderstand ist insbesondere auch ein Stromwiderstand und somit ein niederohmiger elektrischer Messwiderstand. Der Messwiderstand kann insbesondere mit getrennten Strom- und/oder Spannungsklemmen ausgestaltet sein. Der Messwiderstand wird unmittelbar in eine stromführende Leitung eingefügt. Durch ein zu einem solchen Messwiderstand parallelen geschaltetes Spannungsmessgerät wird insbesondere nur ein meist vernachlässigbarer kleiner Strom abgeleitet.

In einer weiteren Ausführungsform des Ultraschallgenerators sind der Kondensator und die mindestens eine Messeinheit derart ausgebildet, dass der Parallelschwingkreis eine Frequenz in einem Bereich von 20 kHz bis 40 kHz, insbesondere von 25 kHz bis 35 kHz, bevorzugt um 27 kHz, aufweist.

Ein "Parallelschwingkreis" (auch "Resonanzkreis" genannt) ist eine resonanzfähige elektrische Schaltung aus einer Spule und/oder Messspule und einem Kondensator, welche elektrische Schwingungen ausführen kann. In dem Parallelschwingkreis wird Energie zwischen dem magnetischen Feld der Spule und/oder Messspule und dem elektrischen Feld des Kondensators insbesondere periodisch ausgetauscht, wodurch abwechselnd hohe Stromstärke oder hohe Spannung vorliegt. Die Resonanzfrequenz f₀ des Parallelschwingkreises berechnet sich nach ${f}_{0} = \frac{1}{2 π \sqrt{L ⋅ C}}$ wobei L für die Induktivität der Spule und C für die Kapazität des Kondensators steht. Wird ein Parallelschwingkreis insbesondere im Bereich seiner Resonanzfrequenz periodisch angeregt, dann führt dieser erzwungene Schwingungen aus. Weist beispielsweise der Kondensator eine Kapazität C von 0,0068 pF und die Spule eine Induktivität L von 0,0093 µH auf, so ergibt sich eine Resonanzfrequenz von 20 kHz. Somit kann die Sinusschwingung erzeugt vom Sinusgenerator mittels des Parallelschwingkreises der Messeinrichtung gemessen und mittels der Steuer- und/oder Regeleinrichtung nachgeregelt werden.

Um die Phasenverschiebung von Spannung und Strom zu bestimmen und die Frequenz nachzuregeln, weist die Steuer- und/oder Regeleinrichtung zwei Komparatoren zum Generieren von Rechtecksignalen aus dem zeitlichen Verlauf des Stroms und der Spannung auf.

Die mittels der beiden Komparatoren generierten Rechtecksignale werden verglichen und die Frequenz der vom Sinusgenerator erzeugten Wechselspannung wird so lange nachgeführt und/oder verändert, bis die Phasenverschiebung zwischen Strom und Spannung Null ist oder einen vorgegebenen Wert erreicht hat. Anstelle der Steuer- und/oder Regeleinrichtung kann auch eine elektronische Abtasteinheit die beiden Komparatoren aufweisen oder eine elektronische Abtasteinheit mit den beiden Komparatoren ist Bestandteil der Steuer- und/oder Regeleinrichtung.

Ein "Komparator" ist insbesondere eine elektronische Schaltung, welche zwei Spannungen vergleicht. Am Ausgang des Komparators steht insbesondere ein Signal zur Verfügung, welches anzeigt, welche der beiden Eingangsspannungen höher ist. Bei einem Komparator kann es sich um einen Schmitt-Trigger und somit um eine elektronische Komparator-Schaltung handeln, bei welcher die Ein- und/oder Ausschaltschwellen nicht zusammenfallen, sondern um eine bestimmte Spannung gegeneinander versetzt sind. Der Schmitt-Trigger dient insbesondere zur Erzeugung von binären Signalen mit steilen Signalflanken und/oder zur Gewinnung von eindeutigen Schaltzuständen aus einem analogen, mit Störeinkopplung belasteten Eingangssignalverlauf. Der Schmitt-Trigger vergleicht insbesondere zwischen einer Eingangsspannung und einer von zwei möglichen Schwellenspannungen. Dadurch wirkt der Schmitt-Trigger als Schwellwertschalter und erzeugt als Ausgangssignal Rechtecksignale bei Überschreiten einer oberen Schwellspannung und bei Unterschreiten einer unteren Schwellspannung. Somit können die Verläufe der Rechtecksignale generiert von den beiden Komparatoren und/oder Schmitt-Triggern bezüglich ihrer Phasenverschiebung analysiert werden.

In einer weiteren Ausführungsform weist der Ultraschallgenerator eine Datenspeicher- und/oder Datenverarbeitungseinheit auf, in welcher eine zuvor bestimmte Frequenz und/oder Resonanzfrequenz der zuordenbaren Sonotrode speicherbar ist oder sind.

Dadurch kann zum einen die bestimmte Resonanzfrequenz der verbundenen Sonotrode mit einer im Ultraschallgenerator hinterlegten Liste von vorhergehenden Resonanzfrequenzen verglichen werden. Zusätzlich kann eine erforderliche oder maximale Leistung bei Verwendung dieser Sonotrode mit einem vorbestimmten Wert ebenfalls in der Datenspeicher- und/oder -verarbeitungseinheit abgelegt sein. Zudem kann durch einen Vergleich der aktuell bestimmten Resonanzfrequenz mit der im Datenspeicher hinterlegten Resonanzfrequenz eine Veränderung und/oder Beschädigung der Sonotrode frühzeitig erkannt und die Sonotrode vor der Anwendung ausgewechselt werden. Vor allem erlaubt das Ablegen der zuvor bestimmten Betriebsfrequenz des Ultraschallgenerators in der Datenspeicher- und/oder Datenverarbeitungseinheit ein Abrufen und Verwenden dieser Frequenz, wenn eine Störung derart groß ist, dass die Messeinrichtung trotz des parallel angeordneten Widerstandes, der Suppressordiode und/oder des Kondensators nicht den zeitlichen Verlauf der Spannung und des Stromes messen und nicht die Phasenverschiebung bestimmen kann und folglich nicht ein Nachregeln der Frequenz mittels des Ultraschallgenerators ermöglicht ist.

Um die Frequenz trotz Störung durch eine Schlaganregung optimal anzupassen und nachzuregeln, weist die Steuer- und/oder Regeleinrichtung eine Phasenregelschleife-Einheit auf, sodass die zuführbare elektrische Leistung und/oder die Frequenz mittels der Steuer- und/oder Regeleinrichtung derart anpassbar ist oder sind, dass eine Phasenverschiebung der zeitlichen Verläufe der Spannung und des Stroms Null ist oder einen vorgegebenen Wert aufweist, und/oder im Falle einer Schlaganregung der Sonotrode mittels des Projektils die zuvor bestimmte Frequenz und/oder Resonanzfrequenz der zuführbaren elektrischen Leistung vorgebbar ist.

Eine "Phasenregelschleife-Einheit" (auch nur "Phasenregelschleife" genannt) ist insbesondere ein Regelkreis mit einem gesteuerten Oszillator, dessen Phase der einen äußeren Signals nachgeführt wird. Bei der Phasenregelschleife ist insbesondere die Abhängigkeit der Stellgröße von der Regelabweichung und somit der Phasenverschiebung periodisch. Die Regelung kann insbesondere auf verschiedene relative Phasenlagen einrasten. Im eingerasteten Zustand ist die Frequenz des Oszillators insbesondere die des Regelsignals.

In einem weiteren Aspekt der Erfindung wird die Aufgabe gelöst durch eine Lithotripsievorrichtung zum Zertrümmern und/oder Entfernen von Körpersteinen, wobei die Lithotripsievorrichtung einen Ultraschallgenerator, eine Trägereinheit mit einem Ultraschallschwingungsanreger und einer distalseitig an der Trägereinheit verbindbaren Sonotrode aufweist, wobei der Ultraschallgenerator elektrisch mit der Trägereinheit verbindbar ist, und der Ultraschallgenerator ein zuvor beschriebener Ultraschallgenerator ist.

Somit wird eine Lithotripsievorrichtung bereitgestellt, in welcher trotz einer elektrischen und/oder mechanischen Störung mittels des Ultraschallschwingungsanregers eine optimale Zertrümmerungsleistung der Sonotrode erzielbar ist.

Um Ultraschallwellen zu erzeugen, weist der Ultraschallschwingungsanreger mindestens ein Piezoelement zwischen einem proximalseitig angeordneten Gegenlager und einem distalseitig angeordneten Horn auf, wobei das mindestens eine Piezoelement mechanisch an das Gegenlager und das Horn gekoppelt ist und das Horn mit einer Halteeinheit der Sonotrode und/oder der Sonotrode verbindbar ist.

Somit ist der Ultraschallschwingungsanreger und/oder das mindestens eine Piezoelement, bevorzugt sind zwei Piezoelemente, in Resonanz mit dem Horn und der Sonotrode betreibbar. Hierbei weist das proximalseitig angeordnete Gegenlager eine Funktion als Reflektor für die von dem Piezoelement oder den Piezoelementen erzeugten Ultraschallwellen auf.

In einer weiteren Ausgestaltungsform ist an dem mindestens einen Piezoelement und/oder zwischen zwei Piezoelement ein elektrisch leitendes Element angeordnet, welches elektrisch mit dem Ultraschallgenerator verbunden ist.

Dadurch kann zum einen mittels der übertragenen Wechselspannung dem Piezoelement oder den Piezoelementen eine mechanische Verformung aufgeprägt werden. Zum anderen kann durch die elektrische Verbindung die spezifische Resonanzfrequenz der Sonotrode mittels der Messeinrichtung des Ultraschallgenerators gemessen werden.

Um eine kombinierte Lithotripsievorrichtung mit einer zusätzlichen Schlaganregung bereitzustellen, weist die Lithotripsievorrichtung ein Beschleunigungsrohr mit einem Hohlraum, einem proximalen Ende, einem distalen Ende und mit einer Längsmittelachse, ein bewegbares Projektil innerhalb des Hohlraum, ein proximalseitiges Anschlagselement, ein distalseitiges Anschlagselement und eine Krafterzeugungseinrichtung zum Hin- und/oder Zurückbewegen des Projektils entlang einer Beschleunigungsstrecke zwischen dem proximalseitigen Anschlagselement und dem distalseitigen Anschlagselement auf, wobei die Sonotrode durch mechanisches Auftreffen des Projektils auf das distalseitige Anschlagselement schwingungsanregbar ist, sodass eine kombinierte Schwingungsanregung der Sonotrode mittels der Krafterzeugungseinrichtung und dem mindestens einen Piezoelement realisierbar ist.

Dadurch kann sowohl die Schwingungsanregung der Sonotrode mittels des Ultraschallschwingungsanregers als auch die Schlaganregung der Sonotrode mittels des Projektils jeweils optimal ausgestaltet und mit maximaler Zertrümmerungsleistung betrieben werden, ohne dass die Schlaganregung mittels des Projektils und die dadurch bedingte Erzeugung von Überspannungen an dem mindestens einen Piezoelement die Messeinrichtung des Ultraschallgenerators stört und/oder eine Nachregelung der Frequenz verhindert.

In einem weiteren Aspekt der Offenbarung wird die Aufgabe gelöst durch ein Verfahren zum Betreiben und/oder Regeln einer Lithotripsievorrichtung zum Vermeiden einer Störung eines Ultraschallgenerators durch ein Aufschlagen eines Projektils, wobei die Lithotripsievorrichtung einen zuvor beschriebenen Ultraschallgenerator aufweist oder eine zuvor beschriebene Lithotripsievorrichtung ist, mit folgenden Schritten:
- Zuführen einer Wechselspannung mit einer Frequenz an einen Ultraschallschwingungsanreger mittels des Ultraschallgenerators, sodass eine Schwingung der Sonotrode mittels des Ultraschallschwingungsanregers angeregt wird,
- Messen von zeitlichen Verläufen einer Spannung und eines Stroms mittels der Messeinheit und Bestimmen einer Frequenz des Parallelschwingkreises der Messeinrichtung,
- Bestimmen der Phasen der zeitlichen Verläufe der Spannung und des Stroms, optional Anpassen der Frequenz mittels einer Phasenregelschleife-Einheit, bis eine Phasenverschiebung der zeitlichen Verläufe der Spannung und des Stroms Null ist oder eine vorgegebene Phasenverschiebung erreicht hat,
- Ablegen der Frequenz in einer Datenspeicher- und/oder Datenverarbeitungseinheit,
- Schlaganregen der Sonotrode mittels eines Projektils und Zuführen einer Wechselspannung mit der zuvor abgelegten Frequenz an den Ultraschallschwingungsanreger, sodass der Ultraschallgenerator frei von einer Störung durch das Aufschlagen des Projektils betrieben wird.

Damit wird ein Verfahren bereitgestellt, mit welchem auf elektronischer Ebene innerhalb des Ultraschallgenerators Störungen verursacht durch das Aufschlagen des Projektils und/oder andere mechanische und/oder elektronische Störungen gedämpft und herausgefiltert werden. Falls trotz Dämpfung und/oder Elimination von Spannungsspitzen dennoch die Messeinrichtung nicht plausible zeitliche Verläufe der Spannung und des Stroms messen kann und folglich keine Phasenverschiebung zur Nachregelung der Frequenz bestimmt werden kann, so wird die zuvor abgelegte Frequenz kurzzeitig während der Störung verwendet, indem im Störungszeitraum eine Wechselspannung mit der zuvor abgelegten Frequenz an den Ultraschallschwingungsanreger zugeführt wird, sodass die Sonotrode optimal mittels des Ultraschallschwingungsanregers schwingungsanregbar ist und ohne dass die Anwendung der Sonotrode durch den Benutzer dadurch beeinträchtigt wird. Das Verfahren ist nicht beansprucht.

Bezüglich des Verfahrens ist herauszustellen, dass dieses das Betreiben und Regeln des Ultraschallgenerators und somit der Lithotripsievorrichtung betrifft. Dies beschränkt sich jedoch auf den internen Betrieb des Ultraschallgenerators und der Lithotripsievorrichtung und umfasst nicht ein Verfahren zur Anwendung der Sonotrode. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Im Weiteren wird die Erfindung anhand von Ausführungsbeispielen näher erläutert. Es zeigen
- Fig. 1: eine stark schematische Darstellung einer Lithotripsievorrichtung mit einem Ultraschallgenerator und einem verbundenen Ultraschallwandler,
- Fig. 2: eine stark schematische Darstellung einer Alternative einer Lithotripsievorrichtung mit einem Ultraschallwandler, einem Beschleunigungsrohr und einem Projektil, und
- Fig. 3: eine schematische Darstellung eines Verfahrens zum Betreiben und Regeln einer Lithotripsievorrichtung.

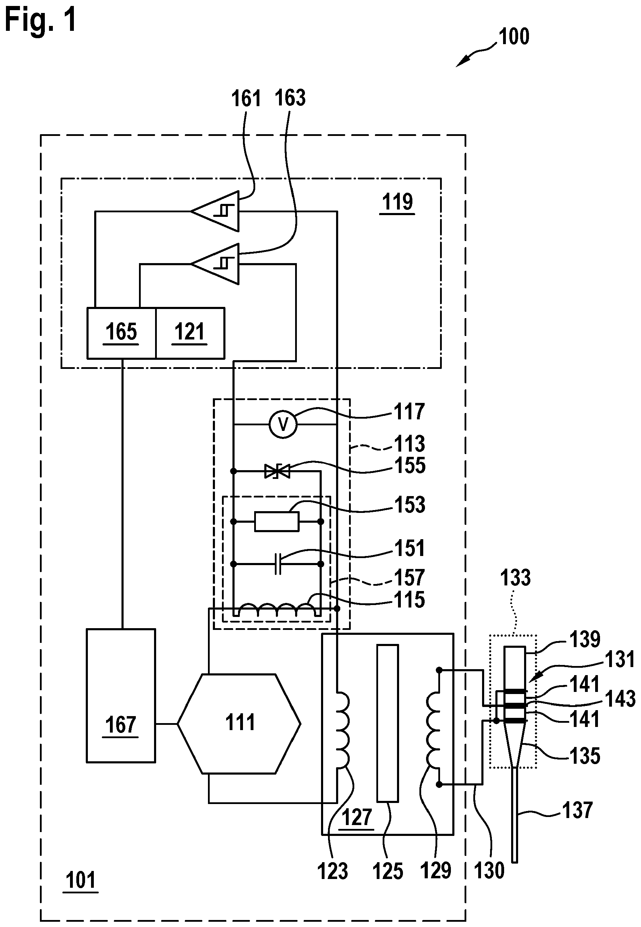

Eine Lithotripsievorrichtung 100 weist einen Ultraschallgenerator 101 und einen Ultraschallwandler 131 auf, welche mittels eines Hochspannungskabels 130 elektrisch miteinander verbunden sind (Fig. 1). Der Ultraschallgenerator 101 weist einen Oszillator 167, einen Sinusgenerator 111, eine Messeinrichtung 113, eine Regeleinrichtung 119, eine Datenverarbeitungseinrichtung 121 und einen Übertrager 127 auf.

Der Ultraschallwandler 131 ist als Handstück 133 ausgebildet. Alternativ kann der Übertrager 127 auch direkt in dem als Handstück 133 ausgebildeten Ultraschallwandler 131 angeordnet sein, sodass das Hochspannungskabel 130 in diesem Fall nicht notwendig ist.

Im Handstück 133 des Ultraschallwandler 131 sind innenliegend proximalseitig ein Gegenlager 139, anschließend zwei Piezoelemente 141 getrennt durch eine Kupferscheibe 143 und distalseitig ein Horn 135 angeordnet. An der distalseitigen Stirnfläche des Hornes 135 ist eine Sonotrode 137 angeordnet.

Ein nicht gezeigtes Netzteil des Ultraschallgenerators 101 weist eine galvanische Trennung zwischen einer primären Netzspannung (90 bis 240 V AC) und einer Kleinspannung auf. Aus der Kleinspannung generiert der Sinusgenerator 111 eine Wechselspannung als Kleinspannung 123 mit einer veränderbaren Amplitude und veränderbaren Frequenz in einem Bereich von 20 bis 34 kHz. Der Übertrager 127 weist eine galvanische Trennung 125 zwischen dieser Wechselspannung als Kleinspannung 123 und einer transformierten Hochspannung 129, wie beispielsweise 400 V, zum Betrieb des Ultraschallwandlers 131 auf, wobei die vom Sinusgenerator 111 eingestellte Frequenz in dem Bereich von 20 bis 34 kHz bei der Übertragung erhalten bleibt. Vor dem Übertrager 127 ist in einer Leitung der Kleinspannung 123 eine Messeinrichtung 113 angeordnet, welche jeweils einen Kanal zur Spannungsmessung 117 und zur Strommessung aufweist. Für die Strommessung weist die Messeinrichtung 113 eine Strommessspule 115 und jeweils parallel geschaltete zur Strommessspule 115 einen Kondensator 151 und einen Widerstand 153 auf, welche zusammen mit der Strommessspule 115 einen Parallelschwingkreis 157 ausbilden. Des Weiteren ist parallel zum Parallelschwingkreis 157 und somit zur Strommessspule 115 eine Suppressordiode 155 angeordnet.

Die Messeinrichtung 113 ist mit der Regeleinrichtung 119 verbunden, über welche die vom Sinusgenerator 111 generierte Wechselspannung 123 mit der veränderbaren Amplitude und/oder Frequenz regelbar ist. Die Regeleinrichtung 119 weist einen ersten Schmitt-Trigger 161 und einen zweiten Schmitt-Trigger 163 auf, welche mit einer Phasenregelschleife-Einheit 165 verbunden sind. Des Weiteren weist die Regeleinrichtung 119 die Datenverarbeitungseinrichtung 121 auf. Die Phasenregelschleife-Einheit 165 ist mit dem Oszillator 167 verbunden, welcher wiederum mi dem Sinusgenerator 111 verbunden ist.

Mit dem Ultraschallgenerator 101 und der Lithotripsievorrichtung 100 werden folgende Arbeitsgänge in einem Verfahren zum Betreiben und Regeln des Ultraschallgenerators 101 und der Lithotripsievorrichtung 100 realisiert:
Vor dem eigentlichen Verfahren wird der Ultraschallwandler 131 und somit die Sonotrode 137 über das Hochspannungskabel 130 mit dem Ultraschallgenerator 101 verbunden. Im ersten Schritt 303 des Verfahrens 301 wird die vom Sinusgenerator 111 generierte Wechselspannung 123 mit einer Frequenz von 27,0 kHz über den Übertrager 127 als Hochspannung 129 den Piezoelementen 141 über die Kupferscheibe 143 zugeführt. Aufgrund der dadurch bedingten Verformung der Piezoelemente 141, welche zwischen dem proximalseitigen Gegenlager 139 und dem distalseitigen Horn 135 eingespannt sind, wird eine Ultraschallschwingung induziert, wodurch ein Anregen einer Resonanzschwingung der verbundenen Sonotrode 137 erfolgt.

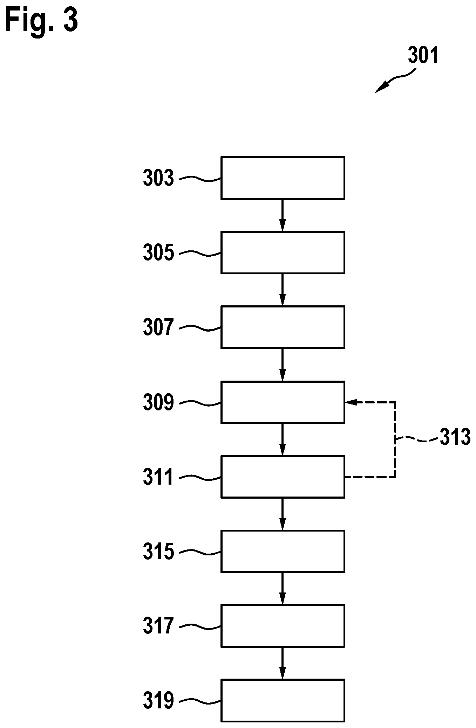

Mittels der Messeinrichtung 113 wird ein kontinuierliches Messen 305 von zeitlichen Verläufen einer Spannung und eines Stroms mittels der Spannungsmessung 117 und der Strommessspule 115 durchgeführt (Figur 3). Gleichzeitig erfolgt ein Bestimmen einer Frequenz des Parallelschwingkreises 157 der Messeinrichtung 113 (Schritt 307). Die gemessenen zeitlichen Verläufe der Spannung und des Stroms werden in der Regeleinrichtung 119 mittels des ersten Schmitt-Triggers 161 und des zweiten Schmitt-Triggers 163 in Rechtecksignale umgewandelt. Anschließend erfolgt ein Bestimmen 309 der Phasen der zeitlichen Verläufe der Spannung und des Stroms mittels der Phasenregelschleife-Einheit 165 und gegebenenfalls Anpassen 311 der Frequenz mittels der Phasenregelschleife-Einheit 165 bis eine Phasenverschiebung der zeitlichen Verläufe der Spannung und des Stroms Null ist und optional ein Wiederholen 313. Die bestimmte Frequenz bei einer Phasenverschiebung von Null wird in der Datenverarbeitungseinrichtung 121 abgelegt (Schritt 315).

Im Falle einer Schlaganregung 317 der Sonotrode 137 durch Auftreffen eines Projektils 271 auf das proximale Ende des Gegenlagers 139 (in Figur 1 nicht gezeigt) oder einer anderen mechanischen und/oder elektronischen Störung wird bei einer dadurch verursachten Überspannung zunächst das rückübertragene Spannungssignal in der Messeinrichtung 113 mittels des Widerstandes 153 gedämpft. Zur weiteren Entstörung ist die Suppressordiode 155 zum Unterdrücken der Überspannung dazugeschaltet, bevor die gemessenen Signale an die Schmitt-Trigger 161, 163 übertragen werden. Wenn die Überspannung derart hoch ist, dass die Messeinrichtung 113 aufgrund der Überlagerung keine sinnvollen Strom- und Spannungswerte messen kann und folglich die Phasenverschiebung nicht bestimmbar ist, erfolgt im nächsten Verfahrensschritt ein Zuführen 319 einer Wechselspannung mit der zuvor bestimmten und abgelegten Frequenz aus der Datenverarbeitungseinrichtung 121 an den Ultraschallwandler 131, sodass der Ultraschallgenerator 101 frei von der Schlaganregung und/oder Störung betrieben wird.

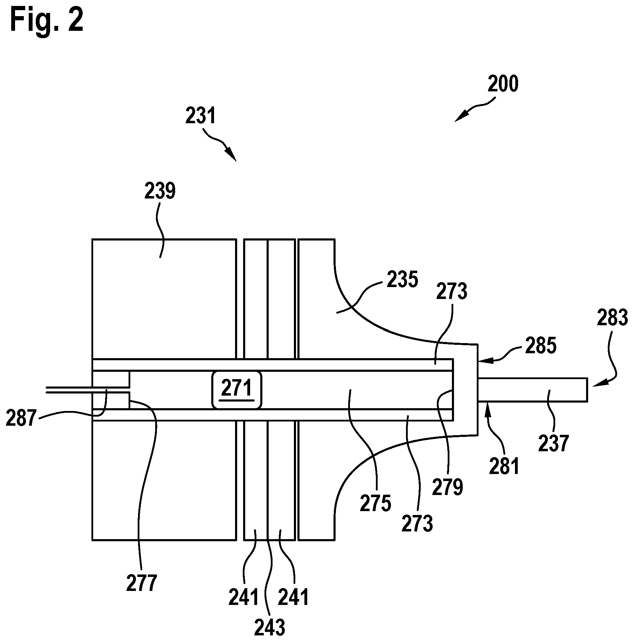

In einer Alternative einer kombinierten Lithotripsievorrichtung 200 weist der Ultraschallwandler 231 ein Gegenlager 239, zwei Piezoelemente 241 mit dazwischen angeordneter Kupferscheibe 243 und ein Horn 235 auf. An einem distalen Ende 285 des Horns 235 ist mit ihrem proximalen Ende 281 eine Sonotrode 237 angeordnet. Das distale Ende 283 der Sonotrode 237 dient zum Zertrümmern von Körpersteinen und ist in der Figur 2 nicht maßstabsgerecht in ihrer Länge dargestellt. Der Ultraschallwandler 231 weist innenliegend einen Hohlraum auf, in dem ein Beschleunigungsrohr 273 angeordnet ist. Innerhalb eines Hohlraums 275 des Beschleunigungsrohrs 273 ist ein Projektil 271 beweglich zwischen einem proximalseitigen Anschlagselement 277 und einem distalseitigen Anschlagselement 279 angeordnet. Proximalseitig des proximalseitigen Anschlagselements 277 ist ein Druckluftzugang 287 zum Einleiten von Druckluft in den Hohlraum 275 zum Bewegen des Projektils 271 angeordnet. Der Ultraschallwandler 231 ist, wie oben beschrieben, mit einem Ultraschallgenerator 101 verbunden. Wie oben beschrieben, werden die Piezoelemente 241 mit einer Hochspannung 129 beaufschlagt und bedingen durch ihre dadurch verursachte Verformung eine Schwingungsanregung der Sonotrode 237. Für eine zusätzliche Schlaganregung wird nun über den Druckluftzugang 287 Druckluft in den Hohlraum 275 des Beschleunigungsrohres 273 gedrückt, wodurch sich das Projektil 271 in Richtung auf das distalseitige Anschlagselement 279 beschleunigt und durch Aufschlagen auf das distalseitige Anschlagselement 279 einen Stoß über das distale Ende 285 des Horns 235 auf das proximale Ende 281 der Sonotrode 237 übertragt, wodurch die Sonotrode 237 zu Schwingung angeregt wird. Da der Anschlag des Projektils 271 auf dem distalseitigen Anschlagselement 279 in unmittelbarer Nähe zu den Piezoelementen 241 erfolgt, wirkt der Schlag auch stark auf die Piezoelemente 241, wodurch diese sich verformen und eine sehr hohe Spannung erzeugen. Diese erzeugte sehr hohe Spannung wird über den Übertrager 127 rückübertragen auf den Ultraschallgenerator 101 und stört die Messeinrichtung 113. In der Messeinrichtung 113 können der Widerstand 153 und die Suppressordiode 155 dieses hohe Spannungssignal nur teilweise dämpfen und unterdrücken. Sobald die Messeinrichtung 113 keine sinnvollen zeitlichen Verläufe von Strom und Spannung mehr bestimmen kann, wird, wie oben beschrieben, eine zuvor in der Datenverarbeitungseinrichtung 121 abgelegte Frequenz über den Oszillator 167 und den Sinusgenerator 111 an den Ultraschallwandler 231 übertragen, sodass der Ultraschallwandler 231 mit einer optimalen Zertrümmerungsleistung der Sonotrode 237 betreibbar ist. Im Weiteren erfolgt das Verfahren zum Betreiben und Regeln des Ultraschallgenerators 101 und der kombinierten Lithotripsievorrichtung 200 wie oben beschrieben.

Somit wird ein Ultraschallgenerator 101 und eine Lithotripsievorrichtung 100, 200 bereitgestellt, bei in einem kombinierten Betrieb mit Schwingungsanregung mittels des Ultraschallwandlers 131, 231 und Schlaganregung mittels des Projektils 271 der Sonotrode 137, 237 beide jeweils mit maximaler Zertrümmerungsleistung betreibbar sind, wobei Störungen durch die Schlaganregung auf die Messeinrichtung 113 des Ultraschallgenerators 101 zunächst durch die Messeinrichtung 113 selbst gedämpft und eliminiert werden und bei einem zu hohen Störungssignal mittels der Regeleinrichtung 119 des Ultraschallgenerators 101 eine zuvor bestimmte Frequenz für den Weiterbetrieb des Ultraschallwandlers 131, 231 an diesen übertragen wird, ohne dass es zu einer Beeinträchtigung der Schwingungsanregung kommt. Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Die Erfindung wird durch die beigefügten Ansprüche definiert.

### Bezugszeichenliste

- 100: Lithotripsievorrichtung
- 101: Ultraschallgenerator
- 111: Sinusgenerator
- 113: Messeinrichtung
- 115: Strommessspule
- 117: Spannungsmessung
- 119: Regeleinrichtung
- 121: Datenverarbeitungseinrichtung
- 123: Kleinspannung (Wechselspannung)
- 125: Galvanische Trennung
- 127: Übertrager
- 129: Hochspannung
- 130: Hochspannungskabel
- 131: Ultraschallwandler
- 133: Handstück
- 135: Horn
- 137: Sonotrode
- 139: Gegenlager
- 141: Piezoelement
- 143: Kupferscheibe
- 151: Kondensator
- 153: Widerstand
- 155: Suppressordiode
- 157: Parallelschwingkreis
- 161: erster Schmitt-Trigger
- 163: zweiter Schmitt-Trigger
- 165: Phasenregelschleife-Einheit
- 167: Oszillator
- 200: Lithotripsievorrichtung
- 231: Ultraschallwandler
- 235: Horn
- 237: Sonotrode
- 239: Gegenlager
- 241: Piezoelement
- 243: Kupferscheibe
- 271: Projektil
- 273: Beschleunigungsrohr
- 275: Hohlraum
- 277: Proximalseitiges Anschlagselement
- 279: Distalseitiges Anschlagelement
- 281: Proximales Ende der Sonotrode
- 283: Distales Ende der Sonotrode
- 285: Distales Ende des Horns
- 287: Druckluftzugang
- 301: Verfahren zum Betreiben und Regeln einer Lithotripsievorrichtung
- 303: Zuführen einer Wechselspannung mit einer Frequenz
- 305: Kontinuierliches Messen von Spannung und Strom
- 307: Bestimmen einer Frequenz des Parallelschwingkreises
- 309: Bestimmen der Phasen
- 311: Anpassen der Frequenz
- 313: Wiederholen
- 315: Ablegen der Frequenz
- 317: Schlaganregen der Sonotrode
- 319: Zuführen einer Wechselspannung mit der zuvor abgelegten Frequenz

## Patentansprüche

1. Ultraschallgenerator (101) zum Zuführen einer elektrischen Leistung zum Zertrümmern von Körpersteinen, wobei dem Ultraschallgenerator (101) eine Sonotrode (137, 237), ein Ultraschallschwingungsanreger (131, 231) zum Anregen einer Schwingung der mindestens einen Sonotrode (137, 237) und eine Krafterzeugungseinrichtung zum Erzeugen einer Kraft zum Bewegen eines Projektils (271) zur Schlaganregung der Sonotrode (137, 237) zuordenbar sind, wobei der Ultraschallschwingungsanreger (131, 231) durch Zuführen einer Wechselspannung (123) mittels des Ultraschallgenerators (101) mit einer Schwingungsfrequenz anregbar ist, und der Ultraschallgenerator (101) eine Messeinrichtung (113) mit mindestens einer Messeinheit (115, 117) zum Messen eines zeitlichen Verlaufs einer Spannung und/oder eines Stroms und eine Steuer- und/oder Regeleinrichtung (119) zum Anpassen einer vom Ultraschallgenerator (101) an den Ultraschallschwingungsanreger (131, 231) zuführbaren elektrischen Leistung aufweist, wobei die Messeinrichtung (113) zumindest einen zu der mindestens einen Messeinheit (115) parallel angeordneten Widerstand (153) und optional einen zur Messeinheit (115) parallel angeordneten Kondensator (151) aufweist, wobei die Messeinrichtung (113) parallel zu der mindestens einen Messeinheit (115) mindestens eine Suppressordiode (155) zum Unterdrücken von Überspannungen aufweist, wobei die Steuer- und/oder Regeleinrichtung (119) eine Phasenregelschleife-Einheit (165) aufweist, sodass die zuführbare elektrische Leistung und/oder die Frequenz mittels der Steuer- und/oder Regeleinrichtung (119) derart anpassbar ist oder sind, dass eine Phasenverschiebung der zeitlichen Verläufe der Spannung und des Stroms Null ist oder einen vorgegebenen Wert aufweist und bei einer Schlaganregung der Sonotrode (137, 237) mittels des Projektils (271) die zuvor bestimmte Frequenz und/oder Resonanzfrequenz der zuführbaren elektrischen Leistung vorgebbar ist.

2. Ultraschallgenerator (101) nach Anspruch 1, **dadurch gekennzeichnet, dass** die mindestens eine Messeinheit eine Messspule (115) ist und die Messeinrichtung (113) den Kondensator (151) zum Ausbilden eines Parallelschwingkreises (157) und den Widerstand (153) zum Dämpfen eines zeitlichen Verlaufes der Spannung und/oder des Stroms aufweist.

3. Ultraschallgenerator (101) nach Anspruch 1, **dadurch gekennzeichnet, dass** die mindestens eine Messeinheit ein Messwiderstand zum Messen des zeitlichen Verlaufs des Stromes ist.

4. Ultraschallgenerator (101) nach Anspruch 1 oder 2, **dadurch gekennzeichnet, dass** der Kondensator (151) und die mindestens eine Messeinheit (115) derart ausgebildet sind, dass der Parallelschwingkreis (157) eine Frequenz in einem Bereich von 20 kHz bis 40 kHz, insbesondere von 25 kHz bis 35 kHz, bevorzugt um 27 kHz, aufweist.

5. Ultraschallgenerator (101) nach einem der vorherigen Ansprüche, **dadurch gekennzeichnet, dass** die Steuer- und/oder Regeleinrichtung (119) zwei Komparatoren (161, 163) zum Generieren von Rechtecksignalen aus dem zeitlichen Verlauf des Stroms und der Spannung aufweist.

6. Ultraschallgenerator (101) nach einem der vorherigen Ansprüche, **dadurch gekennzeichnet, dass** der Ultraschallgenerator (101) eine Datenspeicher- und/oder Datenverarbeitungseinheit (121) aufweist, in welcher eine zuvor bestimmte Frequenz und/oder Resonanzfrequenzen der zuordenbaren Sonotrode (137, 237) speicherbar ist oder sind.

7. Lithotripsievorrichtung (100, 200) zum Zerstrümmern und/oder Entfernen von Körpersteinen, wobei die Lithotripsievorrichtung (100, 200) einen Ultraschallgenerator, eine Trägereinheit (133) mit einem Ultraschallschwingungsanreger (131, 231) und einer distalseitig an der Trägereinheit (133) verbindbaren Sonotrode (137, 237) aufweist, wobei der Ultraschallgenerator elektrisch mit der Trägereinheit (133) verbindbar ist, **dadurch gekennzeichnet, dass** der Ultraschallgenerator ein Ultraschallgenerator (101) nach einem der Ansprüche 1 bis 6 ist.

8. Lithotripsievorrichtung (100, 200) nach Anspruch 7, **dadurch gekennzeichnet, dass** der Ultraschallschwingungsanreger (131, 231) mindestens ein Piezoelement (141, 241) zwischen einem proximalseitig angeordneten Gegenlager (139, 239) und einem distalseitig angeordneten Horn (135, 235) aufweist, wobei das mindestens eine Piezoelement (141) mechanisch an das Gegenlager (139, 239) und das Horn (135, 235) gekoppelt ist und das Horn (135, 235) mit einer Halteeinheit der Sonotrode (137, 237) und/oder der Sonotrode (137, 237) verbindbar ist.

9. Lithotripsievorrichtung (100, 200) nach Anspruch 7 oder 8, **dadurch gekennzeichnet, dass** an dem mindestens einem Piezoelement (141) und/oder zwischen zwei Piezoelementen (141) ein elektrisch leitendes Element (143) angeordnet ist, welches elektrisch mit dem Ultraschallgenerator (101) verbunden ist.

10. Lithotripsievorrichtung (100, 200) nach einem der Ansprüche 7 bis 9, **dadurch gekennzeichnet, dass** die Lithotripsievorrichtung (100, 200) ein Beschleunigungsrohr (273) mit einem Hohlraum (275), einem proximalen Ende, einem distalen Ende und mit einer Längsmittelachse (153), ein bewegbares Projektil (271) innerhalb des Hohlraums (275), ein proximalseitiges Anschlagselement (277), ein distalseitiges Anschlagselement (279) und eine Krafterzeugungseinrichtung zum Hin- und/oder Zurückbewegen des Projektils (271) entlang einer Beschleunigungsstrecke zwischen dem proximalseitigen Anschlagselement (277) und dem distalseitigen Anschlagselement (279) aufweist, wobei die Sonotrode (137, 237) durch mechanisches Auftreffen des Projektils (271) auf das distalseitige Anschlagselement (279) schwingungsanregbar ist, sodass eine kombinierte Schwingungsanregung der Sonotrode (137, 237) mittels der Krafterzeugungseinrichtung und dem mindestens einem Piezoelement (141, 241) realisierbar ist.

## Claims

1. An ultrasonic generator (101) for supplying an electric power for fragmenting body stones, wherein a sonotrode (137, 237), an ultrasonic vibration exciter (131, 231) for exciting a vibration of the at least one sonotrode (137, 237) and a force generating device for generating a force for moving a projectile (271) for impact excitation of the sonotrode (137, 237) are assignable to the ultrasonic generator (101), wherein the ultrasonic vibration exciter (131, 231) is excitable at a vibration frequency by supplying an alternating voltage (123) by means of the ultrasonic generator (101), and the ultrasonic generator (101) has a measuring device (113) with at least one measuring unit (115, 117) for measuring a time profile of a voltage and/or a current and a control and/or regulating device (119) for adapting an electric power which can be supplied from the ultrasonic generator (101) to the ultrasonic vibration exciter (131, 231), wherein the measuring device (113) has at least one resistor (153) arranged in parallel with the at least one measuring unit (115) and optionally has a capacitor (151) arranged in parallel with the measuring unit (115), wherein the measuring device (113) has at least one suppressor diode (155) in parallel with the at least one measuring unit (115) for suppressing overvoltages, wherein the control and/or regulating device (119) has a phase-locked loop unit (165) such that the electric power which can be supplied and/or the frequency can be adapted by means of the control and/or regulating device (119) in such manner that a phase shift of the time profiles of the voltage and the current is zero or has a predefined value and the previously determined frequency and/or resonant frequency of the electric power which can be supplied can be predefined in the case of impact excitation of the sonotrode (137, 237) by means of the projectile (271).

2. The ultrasonic generator (101) according to claim 1, **characterised in that** the at least one measuring unit is a measuring coil (115) and the measuring device (113) has the capacitor (151) for forming a parallel resonant circuit (157) and the resistor (153) for damping a time profile of the voltage and/or the current.

3. The ultrasonic generator (101) according to claim 1, **characterised in that** the at least one measuring unit is a measuring resistor for measuring the time profile of the current.

4. The ultrasonic generator (101) according to claim 1 or 2, **characterised in that** the capacitor (151) and the at least one measuring unit (115) are designed in such manner that the parallel resonant circuit (157) has a frequency in a range from 20 kHz to 40 kHz, in particular from 25 kHz to 35 kHz, preferably around 27 kHz.

5. The ultrasonic generator (101) according to one of the preceding claims, **characterised in that** the control and/or regulating device (119) has two comparators (161, 163) for generating square-wave signals from the time profile of the current and the voltage.

6. The ultrasonic generator (101) according to one of the preceding claims, **characterised in that** the ultrasonic generator (101) has a data storage and/or data processing unit (121) in which a previously determined frequency and/or resonance frequencies of the assignable sonotrode (137, 237) is or are storable.

7. A lithotripsy device (100, 200) for fragmenting and/or removing body stones, wherein the lithotripsy device (100, 200) has an ultrasonic generator, a carrier unit (133) with an ultrasonic vibration exciter (131, 231) and a sonotrode (137, 237) which can be connected to the carrier unit (133) on the distal side, wherein the ultrasonic generator can be electrically connected to the carrier unit (133), **characterised in that** the ultrasonic generator is an ultrasonic generator (101) according to one of claims 1 to 6.

8. The lithotripsy device (100, 200) according to claim 7, **characterised in that** the ultrasonic vibration exciter (131, 231) has at least one piezo element (141, 241) between a proximally arranged counter bearing (139, 239) and a distally arranged horn (135, 235), wherein the at least one piezo element (141) is mechanically coupled to the counter bearing (139, 239) and the horn (135, 235), and the horn (135, 235) can be connected to a holding unit of the sonotrode (137, 237) and/or to the sonotrode (137, 237).

9. The lithotripsy device (100, 200) according to claim 7 or 8, **characterised in that** an electrically conductive element (143) is arranged on the at least one piezo element (141) and/or between two piezo elements (141), which element is electrically connected to the ultrasonic generator (101).

10. The lithotripsy device (100, 200) according to one of claims 7 to 9, **characterised in that** the lithotripsy device (100, 200) has an acceleration tube (273) with a cavity (275), a proximal end, a distal end and with a longitudinal central axis (153), a movable projectile (271) within the cavity (275), a proximal-side stop element (277), a distal-side stop element (279) and a force generating device for moving the projectile (271) back and forth along an acceleration path between the proximal-side stop element (277) and the distal-side stop element (279), wherein the sonotrode (137, 237) is excitable into vibration by mechanical impact of the projectile (271) on the distal-side stop element (279) such that a combined vibration excitation of the sonotrode (137, 237) can be realised by means of the force generating device and the at least one piezo element (141, 241).

## Revendications

1. Générateur d'ultrasons (101) pour la fourniture d'une puissance électrique pour la destruction de calculs dans le corps, dans lequel une sonotrode (137, 237), un excitateur de vibrations ultrasonores (131, 231) pour l'excitation d'une vibration de l'au moins une sonotrode (137, 237) et un dispositif de génération de force pour la génération d'une force pour le déplacement d'un projectile (271) pour l'excitation par percussion de la sonotrode (137, 237) peuvent être associés au générateur d'ultrasons (101), dans lequel l'excitateur de vibrations ultrasonores (131, 231) peut être excité à une fréquence d'oscillation en fournissant une tension alternative (123) par le biais du générateur d'ultrasons (101), et le générateur d'ultrasons (101) présente un dispositif de mesure (113) comportant au moins une unité de mesure (115, 117) pour la mesure d'une évolution dans le temps d'une tension et/ou d'un courant et un dispositif de commande et/ou de régulation (119) pour l'adaptation d'une puissance électrique pouvant être fournie par le générateur d'ultrasons (101) à l'excitateur de vibrations ultrasonores (131, 231), dans lequel le dispositif de mesure (113) présente au moins une résistance (153) agencée en parallèle avec l'au moins une unité de mesure (115) et éventuellement un condensateur (151) agencé en parallèle avec l'unité de mesure (115), dans lequel le dispositif de mesure (113) présente au moins une diode de suppression (155) en parallèle avec l'au moins une unité de mesure (115) pour la suppression de surtensions, dans lequel le dispositif de commande et/ou de régulation (119) présente une unité de boucle à verrouillage de phase (165), de sorte que la puissance électrique pouvant être fournie et/ou la fréquence peuvent être adaptées par le biais du dispositif de commande et/ou de régulation (119) de telle sorte qu'un déphasage des évolutions dans le temps de la tension et du courant est nul ou présente une valeur prédéfinie et que, lors d'une excitation par percussion de la sonotrode (137, 237) par le biais du projectile (271), la fréquence et/ou la fréquence de résonance préalablement déterminée de la puissance électrique pouvant être fournie peut être prédéfinie.

2. Générateur d'ultrasons (101) selon la revendication 1, **caractérisé en ce que** l'au moins une unité de mesure est une bobine de mesure (115) et le dispositif de mesure (113) présente le condensateur (151) pour la conception d'un circuit oscillant parallèle (157) et la résistance (153) pour l'amortissement d'une évolution dans le temps de la tension et/ou du courant.

3. Générateur d'ultrasons (101) selon la revendication 1, **caractérisé en ce que** l'au moins une unité de mesure est une résistance de mesure pour la mesure de l'évolution dans le temps du courant.

4. Générateur d'ultrasons (101) selon la revendication 1 ou 2, **caractérisé en ce que** le condensateur (151) et l'au moins une unité de mesure (115) sont conçus de telle sorte que le circuit oscillant parallèle (157) présente une fréquence dans une plage de 20 kHz à 40 kHz, en particulier de 25 kHz à 35 kHz, de préférence autour de 27 kHz.

5. Générateur d'ultrasons (101) selon l'une des revendications précédentes, **caractérisé en ce que** le dispositif de commande et/ou de régulation (119) comprend deux comparateurs (161, 163) pour la génération de signaux carrés à partir de l'évolution dans le temps du courant et de la tension.

6. Générateur d'ultrasons (101) selon l'une des revendications précédentes, **caractérisé en ce que** le générateur d'ultrasons (101) présente une unité de stockage de données et/ou de traitement de données (121) dans laquelle une fréquence et/ou des fréquences de résonance préalablement déterminées de la sonotrode (137, 237) pouvant être associée peut être stockée ou peuvent être stockées.

7. Dispositif de lithotripsie (100, 200) pour la destruction et/ou l'enlèvement de calculs dans le corps, dans lequel le dispositif de lithotripsie (100, 200) présente un générateur d'ultrasons, une unité de support (133) comportant un excitateur de vibrations ultrasonores (131, 231) et une sonotrode (137, 237) pouvant être connectée du côté distal à l'unité de support (133), dans lequel le générateur d'ultrasons peut être connecté électriquement à l'unité de support (133), **caractérisé en ce que** le générateur d'ultrasons est un générateur d'ultrasons (101) selon l'une des revendications 1 à 6.

8. Dispositif de lithotripsie (100, 200) selon la revendication 7, **caractérisé en ce que** l'excitateur de vibrations ultrasonores (131, 231) présente au moins un élément piézoélectrique (141, 241) entre un contre-palier (139, 239) agencé du côté proximal et un cornet (135, 235) agencé du côté distal, dans lequel l'au moins un élément piézoélectrique (141) est couplé mécaniquement au contre-palier (139, 239) et au cornet (135, 235), et le cornet (135, 235) peut être connecté à une unité de maintien de la sonotrode (137, 237) et/ou à la sonotrode (137, 237).

9. Dispositif de lithotripsie (100, 200) selon la revendication 7 ou 8, **caractérisé en ce qu'**un élément électroconducteur (143) est agencé sur l'au moins un élément piézoélectrique (141) et/ou entre deux éléments piézoélectriques (141), lequel est électriquement connecté au générateur d'ultrasons (101).

10. Dispositif de lithotripsie (100, 200) selon l'une des revendications 7 à 9, **caractérisé en ce que** le dispositif de lithotripsie (100, 200) présente un tube d'accélération (273) comportant une cavité (275), une extrémité proximale, une extrémité distale et un axe central longitudinal (153), un projectile mobile (271) à l'intérieur de la cavité (275), un élément de butée côté proximal (277), un élément de butée côté distal (279) et un moyen de génération de force pour le déplacement du projectile (271) en avant et/ou en arrière le long d'une course d'accélération entre l'élément de butée côté proximal (277) et l'élément de butée côté distal (279), dans lequel la sonotrode (137, 237) peut être excitée de manière vibratoire par l'impact mécanique du projectile (271) sur l'élément de butée côté distal (279), de sorte qu'une excitation vibratoire combinée de la sonotrode (137, 237) peut être réalisée par le biais du dispositif de génération de force et de l'au moins un élément piézoélectrique (141, 241).
